# EUROPÄISCHE PATENTSCHRIFT

(11) **EP 2 589 375 B1**
(45) Veröffentlichungstag und Bekanntmachung des Hinweises auf die Patenterteilung: **26.05.2021**
(21) Anmeldenummer: 12190103.7
(22) Anmeldetag: 26.10.2012
(51) Int. Cl.: A61K 8/97, A61Q 19/08, A61K 8/9789

(54) **VERWENDUNG VON MAGNOLIENRINDENEXTRAKT GEGEN HAUTALTERUNG**
USE OF MAGNOLIA BARK EXTRACT TO COUNTERACT SKIN AGING
UTILISATION D'EXTRAIT D'ÉCORCE DE MAGNOLIA CONTRE LA PEAU MATURE

(30) Priorität: 02.11.2011 DE 102011085591
(43) Veröffentlichungstag der Anmeldung: 08.05.2013
(73) Patentinhaber: Beiersdorf AG, 20253 Hamburg (DE)
(72) Erfinder: Schläger, Torsten, 22297 Hamburg (DE); Holtzmann, Ursula, 22309 Hamburg (DE); Siegner, Ralf, 25421 Pinneberg (DE); Peters, Nils, 22965 Todendorf (DE); Voigt, Nadine, 22111 Hamburg (DE); Winnefeld, Marc, 22880 Wedel (DE); Neufang, Gitta, 20149 Hamburg (DE)

(56) Entgegenhaltungen:
- DE-A1-102010 015 789
- DE-A1-102010 015 790
- JP-A- 2006 328 048
- US-A1- 2008 260 869
- Anonymous: "DaUnBee Activating Liposome Lotion(120ml) from SkinCure, Inc., Korea", , 28. September 2009 (2009-09-28), XP055098178, Gefunden im Internet: URL:http://www.ec21.com/product-details/Da UnBee-Activating-Liposome-Lotion-120ml--63 95960.html [gefunden am 2014-01-23]

## Beschreibung

Die vorliegende Erfindung betrifft kosmetische bzw. dermatologische Zubereitungen, gemäß Anspruch 1 enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Insbesondere nimmt im Alter das subkutane Fettgewebe, insbesondere in der Peripherie (z.B. im Gesicht) ab, so daß es zu typischen Altershauterscheinungen kommen kann.

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar Außerdem sind bisher eine Elastizitätsverbesserung (DE 10 2010 015790 A1 oder US 2008/260869 A1), eine Verbesserung der Mikrozirkulation (DE 10 2010 015790 A1 oder DE 10 2010 015789 A1) oder aber eine allgemeine Verwendung zur Behandlung der Altershaut (JP 2006 328048 A) durch Magnolienrindenextrakte bekannt.

Insbesondere betrifft die vorliegende Erfindung kosmetische Zubereitungen, die imstande sind, das subkutane Fettgewebe der Altershaut zur gesteigerten Fettsynthese zu stimulieren. Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Es hat sich überraschenderweise herausgestellt, daß Magnolienrindenextrakt die Differenzierung von Prä-Adipozyten zu Adipozyten und die Akkumulation von Triglyceriden in Adipozyten anregt.

Magnolien (Magnolia) sind eine Pflanzengattung in der Familie der Magnoliengewächse (Magnoliaceae). Diese enthält etwa 230 Arten, die aus Ostasien und Amerika stammen. Erfindungsgemäß kann der Magnolienrindenextrakt grundsätzlich allen Arten der Gattung Magnolia entnommen werden, wobei allerdings Magnolia grandiflora, und insbesondere Magnolia officinalis bevorzugt werden.

Magnolien-Rinde wird in der Traditionellen Chinesischen Medizin (TCM) vor allem bei "Stagnation of qi" (low energy), emotionalem Stress, Entzündungen, innerer Unruhe und Angstzuständen eingesetzt.

Die Herstellung des verwendeten Extraktes erfolgt mit Hilfe von superkritischem CO2 oder Ethanol oder einer Ethanol / Wasser Mischung.

Magnolol ist durch folgende Struktur gekennzeichnet:

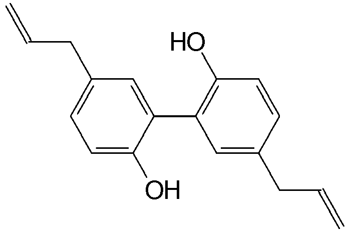

Honokiol ist durch folgende Struktur gekennzeichnet:

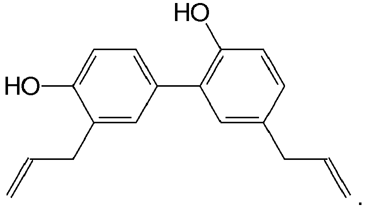

Die erfindungsgemäßen kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an Mangnolienrindenextrakt.

Emulsionen sind vorteilhafte Darreichungsformen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Die nachfolgenden Beispiele sollen die vorliegende Erfindung verdeutlichen.

### Anti-Falten-Creme (Beispiele 1 - 2)

| | **1** | **2** |
|---|---|---|
| **INCI** - **Bezeichnung** | **Gew.-%** | **Gew.-%** |
| Stearinsäure | 2,50 | 2,50 |
| Glycerylstearat | 1,00 | 1,00 |
| C12-15 Alkylbenzoate | 5,00 | 5,00 |
| Caprylsäure/Caprinsäuretriglyceride | 2,50 | 2,50 |
| Cetylalcohol | 2,00 | 2,00 |
| Stearylalcohol | 2,00 | 2,00 |
| Cyclomethicon | 1,00 | 1,00 |
| Dicaprylylcarbonat | 2,00 | 2,00 |
| Dimethicon | 1,00 | 1,00 |
| Glycerin | 5,00 | 5,00 |
| Methylparaben | 0,20 | - |
| Phenoxyethanol | 0,40 | 0,10 |
| Propylparaben | 0,10 | 0,10 |
| Na-disulfit | - | 0,15 |
| Carbomer | 0,10 | 0,10 |
| Acrylate / C10-30 Alkylacrylatcrosspolymer | - | 0,30 |
| Trinatrium EDTA | 0,2 | 0,20 |
| Tapiokastärke | 1,50 | 1,50 |
| Mangnolienrindenextrakt | 0,15 | 0,5 |
| NaOH | q.s. | q.s. |
| Parfum | q.s. | q.s. |
| Wasser | ad 100 | ad 100 |

### Anti-Falten-Creme (Beispiele 3-4)

| | **3** | **4** |
|---|---|---|
| **INCI** - **Bezeichnung** | **Gew.-%** | **Gew.-%** |
| Glycerylstearatcitrat | 2,00 | 3,00 |
| Behenylalcohol | 1,00 | - |
| Glycerylstearat | - | 1,00 |
| C12-15 Alkylbenzoate | 2,50 | 3,00 |
| Caprylsäure/Caprinsäuretriglyceride | 2,50 | 2,00 |
| Cetylalcohol | 2,00 | 2,00 |
| Cyclomethicon | 2,00 | 2,00 |
| Dicaprylylcarbonat | 2,50 | - |
| Dimethicon | 1,00 | - |
| Glycerin | 6,00 | 8,00 |
| Methylparaben | 0,05 | 0,20 |
| Phenoxyethanol | 0,20 | 0,40 |
| Propylparaben | 0,10 | 0,10 |
| Carbomer | 0,10 | 0,20 |
| Natriumpolyacrylat | 0,30 | - |
| Xanthangummi | - | 0,15 |
| Talkum | 1,00 | 1,00 |
| Mangnolienrindenextrakt | 0,50 | 2,00 |
| Pimpinella anisum Extrakt | - | 1,00 |
| Butylmethoxydibenzoylmethan | - | 2,00 |
| Ethylhexylmethoxycinnamate | - | 2,00 |
| Titandioxid | 0,50 | - |
| Octocrylen | 1,00 | 3,00 |
| NaOH | q.s. | q.s. |
| Parfum | q.s. | q.s. |
| Wasser | ad 100 | ad 100 |

### Anti-Falten-Creme (Beispiele 5-6)

| | **5** | **6** |
|---|---|---|
| **INCI** - **Bezeichnung** | **Gew.-%** | **Gew.-%** |
| Polyglyceryl-3 Methylglucosdistearat | 2,50 | 2,50 |
| Sorbitanstearat | 1,00 | 3,00 |
| C12-15 Alkylbenzoate | 2,50 | 2,50 |
| Caprylsäure/Caprinsäuretriglyceride | 2,50 | 2,50 |
| Stearylalcohol | 1,00 | 1,50 |
| Cyclomethicon | 3,00 | 1,00 |
| Dicaprylylcarbonat | 2,50 | - |
| Paraffinum Liquidum | - | 1,00 |
| Dimethicon | - | 1,00 |
| Glycerin | 3,00 | 7,50 |
| Methylparaben | - | 0,20 |
| Phenoxyethanol | 0,10 | 0,20 |
| Propylparaben | 0,10 | 0,10 |
| Carbomer | 0,10 | 0,10 |
| Trinatrium EDTA | - | 1,00 |
| Ammonium Acryloyldimethyltaurat/VP Crosspolymer | 0,25 | - |
| Tapiokastärke | - | 2,50 |
| Talkum | 2,00 | - |
| Creatin | - | 0,75 |
| Creatinin | - | 0,10 |
| Mangnolienrindenextrakt | 0,05 | 1,00 |
| NaOH | q.s. | q.s. |
| Parfum | q.s. | q.s. |
| Ethylhexyl Methoxycinnamate | - | 2,00 |
| Butyl Methoxydibenzoylmethane | - | 2,00 |
| Phenylbenzimidazolsulfonsäure | - | 1,00 |
| Wasser | ad 100 | ad 100 |

### Anti-Falten-Creme (Beispiele 7-8)

| | **7** | **8** |
|---|---|---|
| **INCI** - **Bezeichnung** | **Gew.-%** | **Gew.-%** |
| PEG-40 Stearate | 1,0 | 1,0 |
| Glyceryl Stearate | 2,5 | 3,0 |
| Cera Microcristallina + Paraffinum Liquidum | 2 | - |
| Cetylstearylalkohol | 4 | 3 |
| Caprylsäure/Caprinsäuretriglyceride | 3 | 2,5 |
| Butyrospermum Parkii Butter | 1,5 | - |
| Cyclomethicon | 4 | 2 |
| C12-15 Alkylbenzoate | 2 | 2,5 |
| Glycerin | 2,5 | 7,5 |
| Butylenglykol | 1,5 | 1,0 |
| Mangnolienrindenextrakt | 0,5 | 0,25 |
| Phenoxyethanol | 0,40 | 0,50 |
| lodopropynyl Butylcarbamate | 0,10 | - |
| Methylparaben | - | 0,20 |
| Carbomer | 0,1 | 0,1 |
| Natriumpolyacrylat | - | 0,20 |
| Parfum | q.s. | q.s. |
| Natriumhydroxid | q.s. | q.s. |
| Wasser | Ad 100 | Ad 100 |

## Patentansprüche

1. Verwendung von Magnolienrindenextrakt zur Differenzierung von Prä-Adipozyten zu Adipozyten und zur Anregung der Akkumulation von Triglyceriden in Adipozyten, **dadurch gekennzeichnet, dass** der Magnolienrindenextrakt durch Extraktion der Rinde von Magnolia grandiflora, und/oder Magnolia officinalis mit überkritischem CO₂ oder einem Wasser / Ethanol-Gemisch als Extraktionsmittel gewonnen wird.

2. Verwendung nach Anspruch 1, **dadurch gekennzeichnet, dass** der Magnolienrindenextrakt in kosmetischen oder topischen dermatologischen Zubereitungen in Konzentrationen von 0,001 - 10 Gew.-%, bevorzugt 0,05 - 5 Gew.-%, insbesondere 0,1 - 2,0 Gew.-% bezogen auf das Gesamtgewicht der Zubereitungen, vorliegt.

## Claims

1. Use of magnolia bark extract for differentiating preadipocytes to adipocytes and for stimulating the accumulation of triglycerides in adipocytes, **characterized in that** the magnolia bark extract is obtained by extracting the bark of Magnolia grandiflora and/or Magnolia officinalis with supercritical CO₂ or a water/ethanol mixture as extractant.

2. Use according to Claim 1, **characterized in that** the magnolia bark extract is present in cosmetic or topical dermatological preparations at concentrations of 0.001 - 10% by weight, preferably 0.05 - 5% by weight, especially 0.1 - 2.0% by weight, based on the total weight of the preparations.

## Revendications

1. Utilisation d'extrait d'écorce de magnolias pour la différenciation de pré-adipocytes par rapport à des adipocytes et pour la stimulation de l'accumulation de triglycérides dans des adipocytes, **caractérisée en ce que** l'extrait d'écorce de magnolias est obtenu par extraction de l'écorce de Magnolia grandiflora, et/ou de Magnolia officinalis avec du CO₂ supercritique ou un mélange eau/éthanol en tant qu'agent d'extraction.

2. Utilisation selon la revendication 1, **caractérisée en ce que** l'extrait d'écorce de magnolias est présent dans des préparations cosmétiques ou dermatologiques topiques en des concentrations de 0,001 à 10 % en poids, préférablement 0,05 à 5 % en poids, en particulier 0,1 à 2,0 % en poids par rapport au poids total des préparations.
